# EUROPEAN PATENT SPECIFICATION

(11) **EP 3 053 589 B1**
(45) Date of publication and mention of the grant of the patent: **28.08.2019**
(21) Application number: 14850714.8
(22) Date of filing: 29.09.2014
(51) Int. Cl.: A61K 9/00, A61K 9/06, A61K 47/42, C12N 5/0775, A61K 35/28

(54) **IL-17 PRODUCTION INHIBITORY COMPOSITION**
IL-17-PRODUKTIONS-HEMMENDE ZUSAMMENSETZUNG
COMPOSITION INHIBITRICE DE PRODUCTION DE IL-17

(30) Priority: 02.10.2013 JP 2013207485
(43) Date of publication of application: 10.08.2016
(73) Proprietor: Anterogen Co., Ltd., Geumcheon-gu, Seoul 153-782 (KR)
(72) Inventor: YAMASHITA, Junji, Narita-shi Chiba 286-0825 (JP)
(74) Representative: Ström & Gulliksson AB
(86) International application number: PCT/JP2014/075849
(87) International publication number: WO 2015/050078

(56) References cited:
- WO-A1-2011/070974
- WO-A1-2012/083021
- WO-A2-2010/068707
- WO-A2-2011/047345
- JP-A- 2005 533 480
- JP-A- 2007 020 682
- US-A1- 2005 147 609
- K. LAI ET AL: "Allogeneic adipose-derived stem cells suppress Th17 lymphocytes in patients with active lupus in vitro", ACTA BIOCHIMICA ET BIOPHYSICA SINICA, vol. 43, no. 10, 7 September 2011 (2011-09-07), pages 805-812, XP55273445, US ISSN: 1672-9145, DOI: 10.1093/abbs/gmr077
- ZHANG ET AL: "The effect of allogeneic adipose-derived stem cells on patients with SLE", INTERNATIONAL JOURNAL OF RHEUMATIC DISEASES,, vol. 15, no. SUPPL, 1 September 2012 (2012-09-01), page 27, XP008180266,
- BIN ZHOU ET AL: "Administering human adipose-derived mesenchymal stem cells to prevent and treat experimental arthritis", CLINICAL IMMUNOLOGY, ACADEMIC PRESS, US, vol. 141, no. 3, 26 August 2011 (2011-08-26), pages 328-337, XP028120827, ISSN: 1521-6616, DOI: 10.1016/J.CLIM.2011.08.014 [retrieved on 2011-09-02]
- ZHANG, W. ET AL.: 'The effect of allogeneic adipose-derived stem cells on patients with SLE' INTERNATIONAL JOURNAL OF RHEUMATIC DISEASES vol. 15, no. SUPPL., September 2012, page 27, XP008180266
- LAI, KUAN ET AL.: 'Allogeneic adipose-derived stem cells suppress Th17 lymphocytes in patients with active lupus in vitro' ACTA BIOCHIMICA ET BIOPHYSICA SINICA vol. 43, no. ISSUE, 2011, pages 805 - 812, XP055273445
- ZHOU, BIN; YUAN ET AL.: 'Administering human adipose-derived mesenchymal stem cells to prevent and treat experimental arthritis' CLINICAL IMMUNOLOGY vol. 141, no. 3, 26 August 2011, pages 328 - 337, XP028120827 DOI: 10.1016/J.CLIM.2011.08.014
- ZHOU, YIXUAN ET AL.: 'The therapeutic efficacy of human adipose tissue-derived mesenchymal stem cells on experimental autoimmune hearing loss in mice' IMMUNOLOGY vol. 133, no. 1, 01 May 2011, pages 133 - 140, XP055272551 DOI: 10.1111/J.1365-2567.2011.03421.X
- YUICHI TERAKI ET AL.: 'Possible role of TH 17 cells in the pathogenesisof Stevens-Johnson syndrome and toxic epidermal necrolysis' J ALLERGY CLIN IMMUNOL vol. 131, no. 3, March 2013, pages 907 - 909, XP055273049
- YASUHIKO TOMITA ET AL.: 'Kawasaki Disease -Dai 34 Kai The Society of Kinki Area Kawasaki Disease Research- Kawasaki Disease Arises from the Temporary Dysbiosis of Young Children during the Development of Gut Immune Defense Mechanisms -Speculation on the Etiology of Kawasaki Disease Based on the Literature and Clinical Exper' PROG MED vol. 30, no. 7, 2010, pages 1831 - 1837, XP008180268

## Description

### Field of the Invention

The present invention relates to the IL-17 production inhibitory composition comprising adipose tissue-derived mesenchymal stem cells, in particular, the invention relates to a composition suitable for the prevention or treatment of diseases induced by an increase of IL-17 or Th17 cells.

### Background of the invention

A method for treating damaged or dysfunctional tissue and/or organs, and therapies based on the cells used to multipotent stem cells (cell-based therapy) are generally known as regenerative medicine. Mesenchymal stem cells, one of the multipotent stem cells, can be differentiated to mesenchymal cells lineage (adipocytes, osteoblasts and chondrocytes), muscle cells, nerve cells, endothelial cells, astrocytes and epithelial cells, and they are pluripotent adult stem cells. Therefore, a reconstruction of damaged bones, blood vessels, and myocardium is also expected to, or can be applied to the regenerative medicine and implantation medicine.

Adipose tissue contains a large amount of mesenchymal stem cells. In recent years, extensive studies are being conducted to utilize the adipose tissue-derived mesenchymal stem cells as a transplantation material. The adipose tissue has a considerably large amount of mesenchymal stem cells compared to other tissue types. The adipose tissue has approximately 1,000 times more mesenchymal stem cells compared to the same amount of tissue isolated from the bone marrow. The adipose tissue-derived mesenchymal stem cells as well as the bone marrow-derived mesenchymal stem cells have pluripotency, which can differentiate to chondrocytes, osteoblasts, adipocytes, and muscle cells. Furthermore, the adipose tissue-derived mesenchymal stem cells shows similar cell surface markers expression as bone marrow-derived mesenchymal stem cells, and they also have an immune regulatory activity on the immune response of autologous or allogeneic *in vivo* and *in vitro.*

Interleukin-17A(IL-17A) is a glycoprotein of homodimer consisting of polypeptide with molecular weight of approximately 21 KDa, which was cloned from a murine T cell hybridoma in 1993, and named as a new cytokine (hereinafter just called as IL-17). The IL-17, was found to be produced from helper T cell population (Th17 cells), which is different from Th1 cells and Th2 cells producing IFN-γ and IL-5, respectively. Th17 cells are induced to be differentiated from naive T cells by TGF-β and IL-6 stimulation, and produce IL-17 as well as IL-17F, IL-21, IL-22, IL-26. Findings of the Th17 cells are now able to allow the description of lots of immune phenomena that could not be explained based on the Th1/Th2 theory. In particular, autoimmune diseases such as collagen-induced arthritis and autoimmune encephalomyelitis and the like, which have been considered as Th1-type diseases and they have not been suppressed in IFN-γ or IL-12-deficient mice whereas rather exacerbated. The fact of being strongly inhibited by the IL-17 and IL-23-deficient mice has triggered the concept that Th17 cells may play a very important role in the onset and progression of the pathology of autoimmune diseases. IL-17 induces an inflammatory response, thus it is a major effector cytokines causing the plurality of auto-inflammatory diseases, and its inhibition leads to the development of new effective treatments.

Current treatments for autoimmune diseases have mainly been made via primarily steroid therapy with prednisolone, administration of immunosuppressive agents with cyclosporine, cyclophosphamide and azathioprine, and combination therapy thereof. However, some patients show the side effects of therapeutic agents such as compromised hair loss and osteoporosis, and thus a long-term prognosis is not always satisfactory. Therefore, a new treatment for autoimmune diseases is strongly desired.

In recent years, it has been reported that the human clinical trials with anti-IL-17 antibody has been developed for treating an adaptive inflammatory diseases such as rheumatoid arthritis and psoriasis, however it is not yet commercially available. In addition, the antibody can be used for the purposes of analysis, purification, diagnosis and therapeutic agent because it can be easily prepared, and shows high specificity. However a number of problems has been compromised, for examples, the request for a production system of complex mammalian cells, a dependency on the stability of the disulfide bond, aggregation tendency of antibody fragments, and low solubility still exist. Moreover, the antibodies which are even though designed similarly to the human sequences (humanized antibodies) still have a possibility that they can cause unwanted immune responses, and so it is the most concerned factor/reason in the case of using them as a medicament. In fact, it has been reported that such a non-adaptive case can be occurred due to its immune responses by administering the antibody formulation repeatedly. If the maladaptive symptoms occur, it can be a major problem since such treatment with the therapeutic agent cannot be used any longer.

### Prior Art References

### Non-Patent Literatures

[Non patent literature 1] Harrington LE et al. Interleukin 17-producing CD4+ effector T cells develop via a lineage distinct from the T helper type 1 and 2 lineages. Nat Immunol. 2005 Nov; 6(11): 1123-32.
[Non patent literature 2] Farida Djouad et al. Mesenchymal stem cells: innovative therapeutic tools for rheumatic diseases. Nat Rev Rheumatol. 2009 Jul; 5 (7): 392-9.

### Summary of Invention

### Technical Problem to be Solved

The present invention has been made in view of solving the conventional problems described above. The object of the present invention thereof is to provide a composition for prevention or treatment of the IL-17-related diseases (autoimmune and inflammatory diseases specifically induced by the increase of IL-17).

### Solution to Technical Problem

As a result of intensive studies conducted to achieve the above object, the present inventors found that the adipose tissue-derived mesenchymal stem cells suppress the production of IL-17, and based on the finding, the present invention has been completed.

The present invention has a structure of the following (1) to (4).
1) An IL-17 production inhibitory composition for use in a method for the prevention or treatment of diseases caused by the production of IL-17, wherein the disease is selected from the group consisting of Kawasaki disease, microscopic polyangitis, adult-onset Still's disease, Stevens-Johnson syndrome or toxic epidermal necrolysis, comprising adipose tissue-derived mesenchymal stem cells as an active ingredient, wherein at least 50% of the adipose tissue-derived mesenchymal stem cells are CD10, CD13, CD29, CD44 and CD90 positive, and CD34, CD45 and STRO-1 negative.
(2) The IL-17 production inhibitory composition for use according to (1), wherein it is for injection.
(3) The IL-17 production inhibitory composition for use according to any one of (1)∼(2), wherein the adipose tissue-derived mesenchymal stem cells have been incorporated into Matrigel.
(4) The IL-17 production inhibitory composition for use according to (3), wherein the Matrigel is a fibrin gel.

IL-17 production inhibitory composition of the present invention is able to specifically prevent or treat of IL-17-related diseases induced by the increase of IL-17, because adipose tissue-derived mesenchymal stem cells which is an active ingredient in the composition, suppress the production of IL-17

### Brief Description of Drawings

FIG. 1 shows a diagram of percentage of cells producing IL-17 measured by a flow cytometer, after 3 days of culture under the condition of differentiating from naive CD4 T cells into Th17 cells.
FIG. 2 shows a diagram of the percentage of cells producing IL-17 measured by a flow cytometer. The measurement was performed after three days of culturing under conditions to be differentiated from naive CD4 T cells into Th17 cells, thereafter adding Th17 cells in culture plate of the human and murine adipose tissue-derived mesenchymal stem cells and co-cultured for 1 day.

### Modes for Carrying out the Invention

The present invention is an IL-17 production inhibitory composition containing adipose tissue-derived mesenchymal stem cells as an active ingredient. The adipose tissue-derived mesenchymal stem cells used in the composition of the present invention exhibit a spindle-shaped fibroblast-like shape attached to the culture plate. At least 50 percent of the adipose tissue-derived mesenchymal stem cells, more preferably at least 70% of adipose tissue-derived mesenchymal stem cells as a homogeneous cell population with a positive expression of the stromal cell-associated markers such as CD10, CD13, CD29, CD44 and CD90, but a negative expression of the hematopoietic stem cell associated markers such as CD34, CD45 and STRO-1.

The adipose tissue-derived mesenchymal stem cells used in the compositions of the present invention can be obtained by methods known to those skilled in the art, an example of which is shown below.
(i) A stromal vascular fraction from the adipose tissue (also referred to as SVF) is mainly obtained by liposuction or surgical resection from subcutaneous. Then adipose tissue is separated by treating with collagenase, and centrifuged. After then, the supernatant containing adipocytes is removed, and the suspension is formed by adding a phosphate-buffered saline (also called PBS) to the pellet. Subsequently, the pellet containing the SVF is obtained by centrifugation.
(ii) After the cultivated SVF in the culture medium of the SVF is suspended in culture medium, the suspension is seeded in culture plate at a concentration of 10,000 ∼ 40,000 cells/cm^{2,} and cultured. The culture medium is DMEM (Dulbecco's Modified Eagle Medium) containing 10% fetal bovine serum, which is used to culture the SVF for 24 hours.
(iii) After removing the culture medium of the cultured SVF, the adhesive (or attachment) cells grow by the addition of growth medium. The growth medium is DMEM with a 10% fetal bovine serum and 0.1 ∼ 100 ng/mL of EGF as a growth factor or the material having a similar growth factor activity, which makes the mesenchymal stem cells grow more rapidly and allow them to increase the number of cells significantly in a short period of time.
(iv) When the sub-cultured cells meet 80-90% confluency of the bottom of the culture plate, remove the growth media, trypsinized, and harvested cells from the culture plate. For subculture, the cells are diluted in the ratio of 1: 3 to 1: 4 and then cultured using growth medium in new culture plates. In the same manner as described above, subculture can further be carried out.
(v) By performing such subculture as described above, it is possible to obtain a uniform population of cells (the composition of the present invention) with a positive expression of the stromal cell associated markers such as CD10, CD13, CD29, CD44 and CD90, but a negative expression of the hematopoietic stem cell-associated markers such as CD34, CD45 and STRO-1, etc.

The composition of the present invention can comprise a prophylactically or therapeutically effective amount of adipose tissue-derived mesenchymal stem cells or a cell population of adipose tissue-derived mesenchymal stem cells, and also usually contains pharmaceutically acceptable carriers and / or diluents. Examples of such carriers and diluents are well known to those skilled in the art and, for example, may be mentioned as the followings: lactose, sugars such as glucose and sucrose; starches such as corn starch and potato starch; carboxymethyl cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil, safflower oil, sesame oil, olive oil, corn oil and soybean oils; glycols such as propylene glycol; polyols such as glycerin, sorbitol, mannitol and polyethylene glycol; esters such as ethyl oleate and ethyl laurate; agar; buffering agents such as magnesium hydroxide and aluminum hydroxide; alginic acid; water without comprising pyrogen; isotonic saline; Ringer's solution; ethyl alcohol; pH buffer; polyesters, polycarbonates and/or polyanhydrides; and other non-toxic compatible substances used in pharmaceutical formulations.

The composition of the present invention can contain dimethyl sulfoxide (DMSO) and serum albumin for the purpose of protecting the cells, an antibiotic or the like for the purpose of preventing contamination of bacteria, and various components such as vitamins, cytokines, growth factors, steroids, etc., for the purpose of activating, proliferating or differentiating the cells.

In order to provide the proper administration to the subject, the composition of the present invention comprises a prophylactically or therapeutically effective amount of adipose tissue-derived mesenchymal stem cells, preferably in pure form, with a suitable amount of carriers. As a therapeutically effective amount of cells to be administered, it may be contain, for example, an amount of 1 × 10⁴ ∼ 1 × 10¹⁰ adipose tissue-derived mesenchymal cells in a single dose. Incidentally, the dosage of the adipose tissue-derived mesenchymal cells is determined according to the patient's condition, age and weight, the nature and severity of the disease to be treated or to be prevented, the route of administration, as well as any further treatment formulations. The composition of the present invention may be administered in a single dose or in multiple doses.

Formulations should be suitable for the mode of administration. The composition of the present invention is provided in a sterile condition as a preparation without comprising pyrogen for the administration of the composition of the present invention to human subjects, which is suitable for any routes of administration and is not particularly limited. For example, it can be administered topically, orally, parenterally by inhalation spray, rectally, nasally, transbuccally, or vaginally. It also can be administered over an eye or an implanted reservoir. The term "parenterally" used above includes subcutaneous, intradermal, intravenous, intramuscular, intra-articular, intrasynovial, intrasternal, intrathecal, intralesional and intracranial injection, or infusion techniques.

In the composition of the present invention, the adipose tissue-derived mesenchymal stem cells can be used after being incorporated into the cell carrier (Matrigel). Thus, it is possible to be effectively administered and activated in therapeutic indications topically. It may be good for Matrigel to embed the cells and the Matrigel may be, for example, fibrin gel (fibrin glue), hyaluronic acid, alginic acid, polylactic acid, and glycolic acid. Furthermore, it is useful to use the Matrigel with extracellular matrix such as laminin, collagen IV, entactin or Matrigel supplemented with extracellular substrates supplemented with the growth factors. The Matrigel is mixed with adipose tissue-derived mesenchymal stem cells, and is applied after layering or mixing in therapeutic indications site. The Matrigel to be mixed with adipose tissue-derived mesenchymal stem cells is preferably fibrin gel. Fibrin gels are prepared by mixing fibrinogen and thrombin, for example, are for Boruhiru tissue adhesion (chemical and serum therapy Kenkyusho). A mixture with adipose tissue-derived mesenchymal stem cells and Matrigel increases or decreases appropriately according to the size, and so it is applied to the affected area (bonding and closure site) after layering or mixing.

The composition of the present invention has IL-17 production inhibitory effect, and so it can prevent or treat the IL-17-related diseases caused by the increase of IL-17. For example, IL-17-related diseases can include, but are not limited to, Kawasaki disease, microscopic polyangitis, adult-onset Still's disease, Stevens-Johnson syndrome, and toxic epidermal necrolysis.

### Description of Embodiments

Examples of the preparation, effect, and application of the composition of the present invention are described below. The present invention is not limited thereto.

### (Example 1)

Human adipose-derived mesenchymal stem cells preparation Subcutaneous fatty tissue was obtained from normal human by liposuction. In order to remove the blood from the fat tissue, the obtained adipose tissue was washed with an equal volume of PBS. The equal volume of collagenase solution was added to the adipose tissue and was digested at 37°C so that fat layer was eliminated. After digestion, it was centrifuged and the pellet containing the SVF was obtained after removing the supernatant containing fat cells. The SVF culture medium (DMEM, 10% FBS, antibiotics) was suspended and seeded in culture plates, 37°C, at 5% CO₂ incubator and cultured for about 24 hours. Thereafter, the floating cells such as blood cells were removed by washing with PBS, the adipose tissue-derived mesenchymal stem cells having adhesive ability, were selected as cells adhered to the culture plate. Thereafter, we had extended culture of the adipose tissue-derived mesenchymal stem cells in the growth medium (DMEM, 10% FBS, 1ng / mL bFGF). After the adipose tissue-derived mesenchymal stem cells proliferated until the cells cover up to 80% of the culture plates, the cells were detached by trypsinization, and the resulting cells were diluted with a growth medium in the ratio of 1: 3 to 1: 4 and the subculture was repeated to three or four passages. For cell suspension after subculture, the sub-cultured cells were filled into vials to prepare a human adipose tissue-derived mesenchymal stem cell suspension. This cell suspension, more than 80 percent of the adipose tissue-derived mesenchymal stem cells were positive to CD10, CD13, CD29, CD44 and CD90, and negative to CD34, CD45 and STRO-1.

### (Example 2)

The subcutaneous adipose tissue was obtained from 6-week-old female C57BL/6 mice for preparation of tissue-derived mesenchymal stem cell. The subcutaneous adipose tissue was added in an equal volume of collagenase solution and was digested at 37°C to eliminate fat layer. After digestion, it was centrifuged and the pellet containing the SVF was obtained after removing the supernatant containing fat cells. The SVF culture medium (DMEM, 10% FBS, antibiotics) was suspended and seeded in culture plates at 37°C, in 5% CO₂ incubator and cultured for about 24 hours. Thereafter, the floating cells such as blood cells were removed by washing with PBS, since the adipose tissue-derived mesenchymal stem cells having adhesion ability. The cells adhered to the culture plate were selected as adipose tissue-derived mesenchymal stem cells. After that, expansion of the adipose tissue-derived mesenchymal stem cells was performed in the growth medium. The adipose tissue-derived mesenchymal stem cells were cultivated until the cells reached 80% confluency of the culture plates, the cells were detached by trypsinization, and the resulting cells were diluted with a growth medium in the ratio of 1: 3 to 1: 4 and the subculture was repeated until three or four passages. For cell suspension after subculture, the sub-cultured cells were filled into vials to prepare a murine adipose tissue-derived mesenchymal stem cell suspension. This cell suspension, more than 70 percent of the adipose tissue-derived mesenchymal stem cells showed a positive response to CD29 and CD90, and a negative response to CD34 and CD45.

### (Example 3)

Inhibitory effect of IL-17-producing cells by human and murine adipose tissue-derived mesenchymal stem cells (1).

### Th17 cells preparation

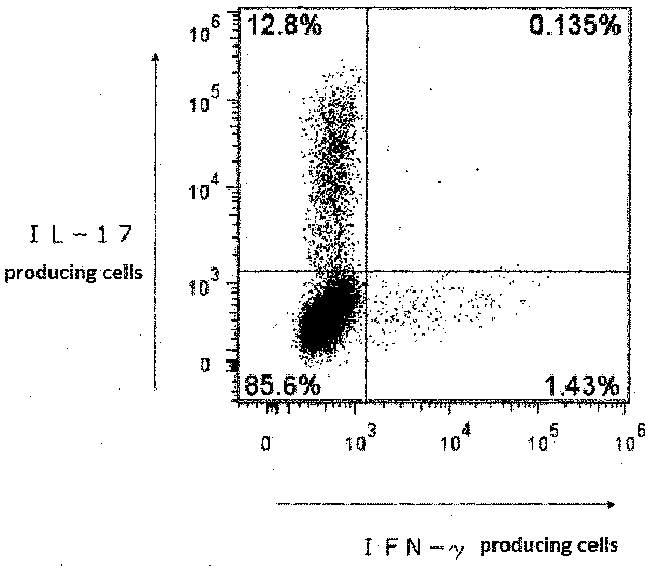

Female C57BL/6 mice (6-week-old Japan Charusuriba)'s lymphocytes were isolated from cervical lymph nodes. After that, naive CD4 T cells were separated using the CD4⁺ CD62L⁺ T Cell Isolation Kit II (130-093-227 MiltenyiBiotec Inc.). Then, 1.5 x 10⁶ of naive CD4 T cell were seeded in 24 wells plate which was coated with murine anti-CD3 antibody (16-0031; eBioscience). The naive CD4 T cells were incubated for 3 days (37°C, 5% CO₂) to induce differentiation from naive CD4 T cells into Th17 cells under conditions to be (IL-6; 50ng / mL; BioLegend, TGF- β; 1ng / mL; BioLegend, IL-23; 5ng / mL; BioLegend, antiIL-4 antibody; 10µg / mL; BioLegend, anti IFN-γ antibody; 10µg / mL; BioLegend, anti-CD28 antibody; 5µg / mL; BioLegend). After 3 days in culture, the cells were stained with anti-murine IL-17 antibody (12-7177-81; eBioscience) and anti-murine IFN-γ antibody (17-7311 eBioscience). The ratio of naive CD4 T cells to Th17 cells were measured with a flow cytometer (Cytomics FC500; BECKMAN COULTER) (Figure 1).

As shown in Figure 1, the percentage of cells producing IL-17 after 3 days of culture under the condition of differentiating into Th17 cells was 12.80%. Meanwhile, the cells producing IFN-γ was 1.43%, which revealed that it is differentiated from naive CD4 cells into Th17 cells.
(2) After the naive T cells were being cultured for 3 days under the condition of differentiating the naive T cells into the Th17 cells, and we obtained Th17 cells which can produce the IL-17 (Figure 1). We added 1.0 X 10⁶ Th17 cells to the 24 well plate that reached 80% confluency of human and murine adipose tissue-derived mesenchymal stem cells (1.0 X 10⁴ cells) which were produced by examples 1 and 2 and then co-cultured for 1 day.

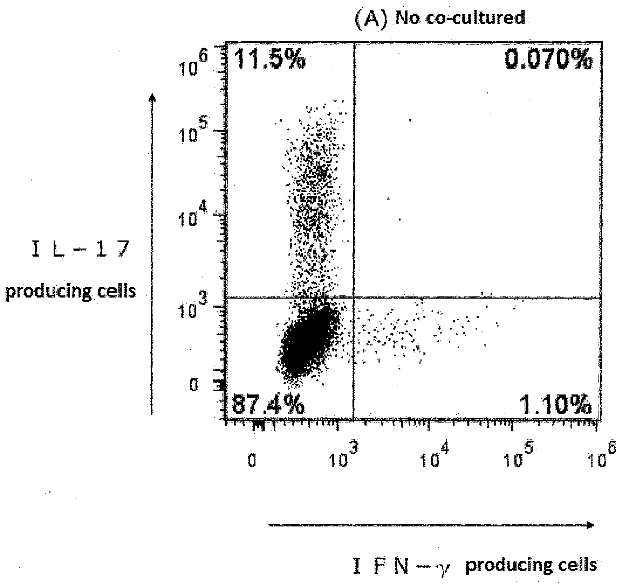

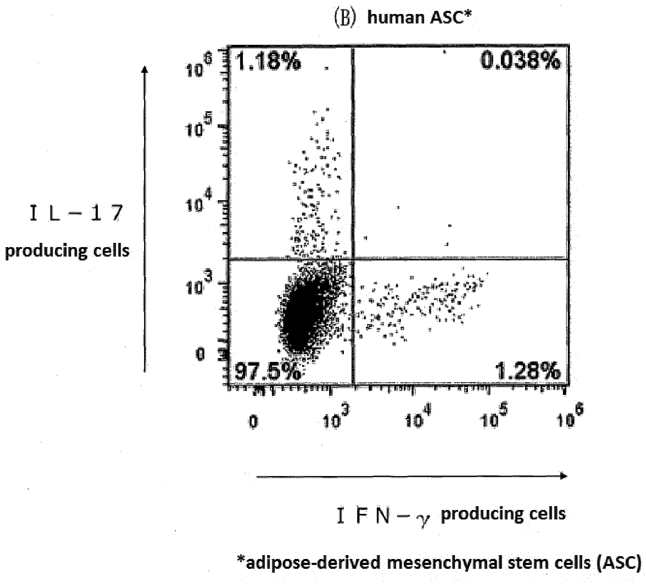

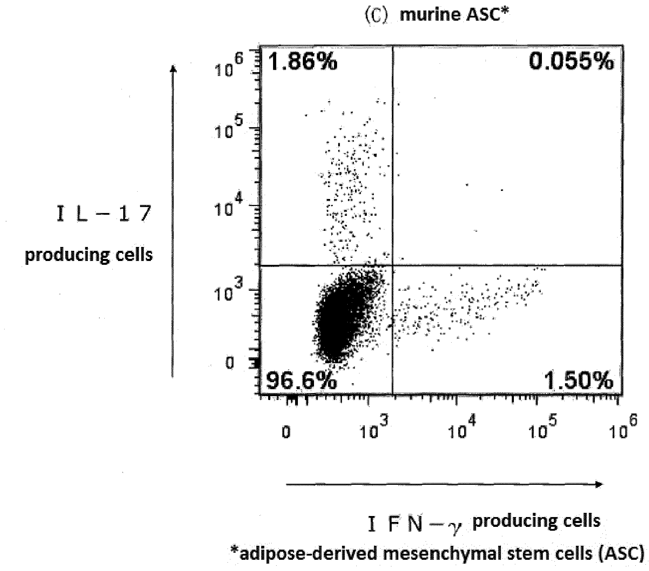

After co-culture, the Th17 cells were prepared and stained with anti-IL-17 antibody and anti-IFN-γ antibody, and the percentage of Th17 cells that produce IL-17 was measured by flow cytometer (Figure 2 and Table 1).

As shown in Figure 2, without co-culture (A), the percentage of cells producing IL-17 was 11.5%. The addition of Th17 cells in a culture plate of the human adipose tissue-derived mesenchymal stem cells (B), the percentage of cells producing IL-17 was 1.18%. The addition of Th17 cells in a culture plate of murine adipose tissue-derived mesenchymal stem cells (C), the percentage of cells producing IL-17 was 1.86%. On the other hand, the percentage of cells that produce IFN-γ, was not major changed in each group.

**[Table 1]**

| | (A) No ASC | (B) human ASC* | (C) murine ASC* |
|---|---|---|---|
| Inhibition rate of IL-17 producing cells(%) | 0% | 89.6% | 93.5% |

| | | | |
|---|---|---|---|
| *adipose-derived mesenchymal stem cells(ASC) | | | |

As shown in Table 1, inhibition rate of IL-17 was 89.6% in case (B), which Th17 cells were added to the culture plate with human adipose tissue-derived mesenchymal stem cells, and in case (C), which Th17 cells were added to the culture plate with murine adipose tissue-derived mesenchymal stem cells, inhibition rate of IL-17 producing cells was 93.5%.

From the above, the human and murine adipose tissue-derived mesenchymal stem cells were revealed to inhibit IL-17-producing cells significantly.

### (Example 4)

Human adipose tissue-derived mesenchymal stem cell suspensions were prepared in Example 1 as the external medicine of the adipose tissue-derived mesenchymal stem cells was included in Boruhiru tissue adhesion by the following manner. That is, the fibrinogen lyophilized powder (vial 1) was dissolved in the total amount of fibrinogen solution (vial 2) to obtain a solution A (fibrinogen concentration; 80 mg / mL).

Thrombin lyophilized powder (vial 3) was dissolved in the total amount of thrombin solution (vial 4) to obtain a solution B (thrombin concentration; 250 units). After diluting the adipose tissue-derived mesenchymal stem cells in solution A by mixing with the equivalent amount of liquid B, we produced external remedies of adipose tissue-derived mesenchymal stem cells.

### (Example 5)

C57BL/6 mice (4 week old, male) were intraperitoneally injected with 0.5 mg of LCWE (Lactobacillus casei cell wall extract) to induce Kawasaki disease and elucidated for the improvements of 4-week-old human adipose tissue-derived mesenchymal stem cells in Kawasaki disease model mice. Human adipose tissue-derived mesenchymal stem cells of 5× 10⁵ from Example 1, after preparation to 2 × 10⁶ cells per 1 mL in PBS, was administered intravenously after two weeks of LCWE administration. The mice was euthanized at age of 8 weeks, aortic root including the side coronary bifurcation were extracted and the histopathological examination of coronary artery inflammation was performed in each group.

Compared with the negative control group, the result of cellular infiltration surrounding ranges and coronary lesions in human adipose tissue-derived mesenchymal stem cells administered group was significantly suppressed.

### Industrial Applicability

Since the composition of the present invention has an effect of inhibiting the production of IL-17, it will be effectively used in preventing or treating IL-17-related diseases caused by the production of IL-17.

## Claims

1. An IL-17 production inhibitory composition for use in a method for the prevention or treatment of diseases caused by the production of IL-17, wherein the disease is selected from the group consisting of Kawasaki disease, microscopic polyangitis, adult-onset Still's disease, Stevens-Johnson syndrome or toxic epidermal necrolysis, comprising adipose tissue-derived mesenchymal stem cells as an active ingredient, wherein at least 50% of the adipose tissue-derived mesenchymal stem cells are CD10, CD13, CD29, CD44 and CD90 positive, and CD34, CD45 and STRO-1 negative.

2. The IL-17 production inhibitory composition for use according to claim 1, wherein it is for injection.

3. The IL-17 production inhibitory composition for use according to any one of claims 1 or 2, wherein the adipose tissue-derived mesenchymal stem cells have been incorporated into Matrigel.

4. The IL-17 production inhibitory composition for use according to claim 3, wherein the Matrigel is a fibrin gel.

## Patentansprüche

1. IL-17-Produktionshemmungszusammensetzung zur Verwendung bei einem Verfahren zur Prävention oder Behandlung von Krankheiten, die durch die Produktion von IL-17 verursacht werden, wobei die Krankheit ausgewählt ist aus der Gruppe, die Kawasaki-Syndrom, mikroskopische Polyangiitis, adulten Morbus Still, Stevens-Johnson-Syndrom oder toxische epidermale Nekrolyse umfasst, aufweisend aus Fettgewebe gewonnene mesenchymale Stammzellen als aktive Zutat, wobei mindestens 50% der aus Fettgewebe gewonnenen mesenchymalen Stammzellen CD10-positiv, CD13-positiv, CD29-positiv, CD44-positiv, CD90-positiv, CD34-negativ, CD45-negativ und STRO-1-negativ sind.

2. IL-17-Produktionshemmungszusammensetzung zur Verwendung nach Anspruch 1, wobei sie zur Injektion vorgesehen ist.

3. IL-17-Produktionshemmungszusammensetzung zur Verwendung nach einem der Ansprüche 1 oder 2, wobei die aus Fettgewebe gewonnenen mesenchymalen Stammzellen in Matrigel aufgenommen wurden.

4. IL-17-Produktionshemmungszusammensetzung zur Verwendung nach Anspruch 3, wobei das Matrigel ein Fibringel ist.

## Revendications

1. Composition inhibitrice de production d'IL-17 destinée à être utilisée dans une méthode pour la prévention ou le traitement de maladies provoquées par la production d'IL-17, dans laquelle la maladie est sélectionnée dans le groupe constitué de la maladie de Kawasaki, de la polyangéite microscopique, de la maladie de Still de l'adulte, du syndrome de Stevens-Johnson ou d'une nécrose épidermique toxique, comprenant des cellules souches mésenchymateuses dérivées de tissu adipeux en tant que principes actifs, dans laquelle au moins 50 % des cellules souches mésenchymateuses dérivées de tissu adipeux sont CD10, CD13, CD29, CD44 et CD90 positives, et CD34, CD45 et STRO-1 négatives.

2. Composition inhibitrice de production d'IL-17 destinée à être utilisée selon la revendication 1, dans laquelle ladite composition est destinée à une injection.

3. Composition inhibitrice de production d'IL-17 destinée à être utilisée selon l'une ou l'autre de la revendication 1 ou 2, dans laquelle les cellules souches mésenchymateuses dérivées de tissu adipeux ont été incorporées dans du Matrigel.

4. Composition inhibitrice de production d'IL-17 destinée à être utilisée selon la revendication 3, dans laquelle le Matrigel est un gel de fibrine.
